# EUROPEAN PATENT APPLICATION

(11) **EP 3 865 471 A1**
(43) Date of publication of application: **18.08.2021**
(21) Application number: 20156678.3
(22) Date of filing: 11.02.2020
(51) Int. Cl.: C07C 217/94, C07C 245/20, C07D 333/50, C08J 7/04, A61K 47/58, A61K 47/62, A61K 47/69

(54) **METAL-BASED NANOMATERIALS COATED WITH CALIXARENES**

(71) Applicant: Université Libre de Bruxelles, 1050 Bruxelles (BE)
(72) Inventor: BRUYLANTS, Gilles, 1200 Bruxelles (BE); JABIN, Ivan, 1180 Bruxelles (BE); RETOUT, Maurice, 1050 Bruxelles (BE)
(74) Representative: Office Kirkpatrick

(57) **Abstract**

This invention concerns a versatile and simple one-pot method to prepare nanomaterials, and in particular nanoparticles, grafted with an ultra-thin layer of calixarenes by placing at least one oxidized metal with at least one calix[n]arene diazonium salt in the presence of a reducing agent in a solvent, and heating the reaction mixture to obtain a metal-based nanomaterial coated with calix[n]arenes. The invention further concerns the use of these calixarene-grafted nanomaterials to further functionalize the surface of the nanomaterials.

## Description

### Field of the Invention

This invention concerns a versatile and simple one-pot method to prepare nanomaterials, and in particular nanoparticles, grafted with an ultra-thin layer of calixarenes as well as a new process for manufacturing these materials. The invention further concerns the use of these calixarene-grafted nanomaterials.

### Background of the Invention

The use of nanomaterials, and in particular metal-based nanomaterials has become widespread in many technical fields, from biomedical applications or paints to microelectronics or as chemical catalysts. Their preparation is however not always straightforward, due to stability issues or difficulty to control their size and/or shape. Their functionalization is not simpler for the same stability reasons, and because reactive groups have to be introduced at the surface of the nanomaterial.

For example, silver nanoparticles are known to have anti-bacterial effect, which in part results from their instability and release of silver ions. This same instability prevents them from being functionalized. It is therefore so far extremely difficult to prepare functionalized silver nanoparticles.

One class of organic substances that has been proposed for immobilizing or grafting onto material surfaces is that of the calix[n]arenes (or calixarenes). Calix[n]arenes are cyclic phenoxy derivatives in which n is the number of phenoxy groups, linked in their ortho positions by methylene bridges or sulfur bridges in the case of thiacalixarenes. Calixarenes are conformationally flexible molecules that can display different conformations due to the ability to undergo complete ring inversions. Calixarenes can eventually possess a cup-like structure (i.e. cone conformation) having a narrow and a large rim.

In the following, the terms "calix[n]arenes" and "calixarenes" refer to both families of compounds, those with methylene bridges (named calix[n]arenes), and those with S bridges (named thiacalix[n]arenes) and their oxide derivatives (SO and SO₂ bridges). In addition, calix[n]arenes also have varying numbers of phenoxy moieties expressed by the symbol [n], wherein n represents the number of phenoxy moieties, in particular n can be 4, 5, or 6. Calix[n]arenes are known compounds that have been synthesized with various substitution patterns, for example with substituents on the aromatic part of the phenoxy moieties or on their hydroxyl groups. These cyclic compounds can find various applications in a manifold of areas, including the development of enzyme mimetics, ion sensitive electrodes or sensors, selective membranes, non-linear optics, and HPLC stationary phases.

Calix[n]arenes have been used as coatings on various materials. The immobilization of (thia)calixarenes onto a surface has been reported using different attachment techniques. The resulting immobilized calixarenes were used as receptors.

EP2836539 discloses a grafting method to coat an ultrathin layer of calixarenes on the surface of a material. The calixarenes are grafted via the large rim, using diazonium chemistry, and it leads to rigid and stable molecular layers, which offer a robust and stable platform for further (post-)functionalization. The macrocyclic structure of the calixarene prevents polymerization during the grafting process, induces spatial pre-organization and pre-structuration and allows the orthogonal polyfunctionalization of the platforms, with a precise spatial control.

Though leading to robust grafting, this method can only be efficient for stable nanomaterials, and could, for example not be applied to silver nanostructures.

There is a need for a more versatile and tunable method to prepare nanomaterials functionalized with calixarenes, with a highly robust, structurally regular, ultra-thin layer, in a controlled manner. Indeed, for certain applications, it is important to control the size and shape of the nanomaterial as well as the surface density, e.g. for applications as sensors, because this may have an impact on the detection sensitivity and efficiency.

### Summary of the Invention

In accordance with the present invention, a one-pot synthesis gives nanomaterials grafted or coated with an ultra- thin layer of calix[n]arenes from a metallic salt (or a mixture of metallic salts) and a calixarene-diazonium salt (or a mixture of calixarene-diazonium salts), in reductive conditions. This leads to the formation of a robust nanomaterial coated with an ultra-thin layer, which may be rather dense and which, if desired, can serve as a platform for one or more further functionalization(s).

To this purpose, the invention relates to a method to synthesize metal-based nanomaterials coated with calix[n]arenes comprising the steps of:
- placing at least one oxidized metal MX with at least one calix[n]arene-diazonium salt C-N₂ in presence of a reducing agent in a solvent, and
- heating the reaction mixture to obtain a metal-based nanomaterial coated with calix[n]arenes MC.

By at least one, it is referred to at least one type of.

An oxidized metal refers to a metal with metal or metal derivative having a non-null oxidation state. Preferably, an oxidized metal refers to a metal having a positive oxidation state. It can be a metal salt or a metal oxide.

The method offers access to robust and stable nanomaterials, which, depending on the type of calixarene used, can be further functionalized. The macrocyclic structure of the calixarene prevents polymerization during the synthesis process, induces spatial pre-organization and pre-structuration and allows the orthogonal polyfunctionalization of the platforms, with a control on the composition of the final layer.

The reaction scheme is as follows: 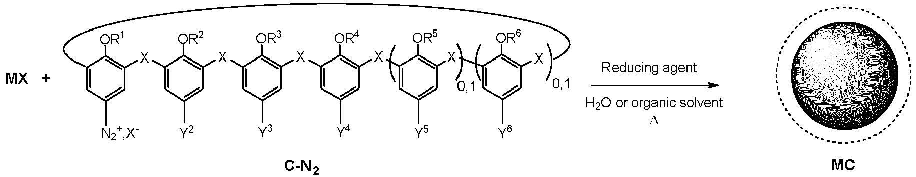 Using this method, the calix[n]arenes are bound onto the metallic surface of the nanomaterial with covalent bonds.

The method of the invention can further comprise the step of adjusting the value of the pH of the reaction mixture preferably below 8, preferably below 7.5, still preferably below 7 and above 5, preferably above 5.5 and still preferably above 6, preferably around 6.5.

The molar ratio of metal-based salt to calixarenes influences the number of particles formed and hence their size, as well as the quality of the coating. A too large excess of calixarene diazonium may lead to the calixarene reacting with itself. This ratio should preferably be fixed between 5:1 and 1:5, preferably between 4:1 and 1:4, preferably between 2:1 and 1:2, still preferably around 1:1.

The reducing agent can be any reducing agent found suitable by the person skilled in the art like for example hydrides (e.g. sodium borohydride, sodium cyanoborohydride, ...), ascorbate salts (e.g. sodium ascorbate), etc. The strength and the amount of reducing agent can be modified in order obtain different sizes: weaker reducing agents or lower amounts of it will lead to bigger nanoparticles. The molar proportion of reducing agent to calixarene-diazonium salt is preferably comprised between 20:1 and 1:10, preferably between 15:1 and 1:5, preferably between 10:1 and 1:4, preferably between 5:1 and 1:2, still preferably around 4:1. The person skilled in the art will know how to adapt proportions, taking for example into account the number of hydrides a given reducing agent can provide.

Heating is preferably performed under mixing, at temperatures comprised between 25 °C and 150 °C, preferably between 30 and 120 °C, preferably between 40 °C and 100 °C, preferably between 50 and 80 °C and still preferably around 60 °C. It was observed by the applicants that the reaction temperature has an impact on the particle size distribution. A reaction temperature of around 60°C leads to a narrow size distribution.

Reaction time is at least 1h, preferably at least 2h or at least 3h, still preferably at least 5h or at least 8h and up to 48h, up to 24h, preferably up to 16h. The applicant has noted that after 16h, the formation of the nanoparticles was completed as well as the grafting of the calixarenes onto these particles.

The invention also relates to the metal-based nanomaterials coated with calix[n]arenes obtained by the process of the invention, which have the distinctive features over nanomaterials obtained by other methods to have an homogeneous particles distribution (i.e. above 80% of the particle formed have the same average dimension ± 10%). Moreover, the metal-based nanomaterials of the invention are coated only with calix[n]arenes, meaning that the only ligands at the surface of the metal are calixarenes. Indeed, with other methods, where a ligand like citrate, present at the surface of a particle, is interchanged with a calixarene, there are always some residual citrate ligands at the surface of the metal. The metal-based nanomaterials of the invention can therefore be distinguished from metal-based nanomaterials prepared by ligand-exchange techniques by the absence of residual ligands other than calixarenes at their surface.

Preferably, the oxidized metal MX is a metal oxide or a mixture of metal oxides, or a salt of a metal or a mixture of salts.
The salt generally implies that the metallic part is positively charged, the counter ion can be any suitable ion. The metal-based salt can for example be a halide, a nitrate, a sulfate, a carboxylate, a triflate, an alcoolate, an hydroxylate, a sulfonate, a phosphate (like hexafluorophosphate), a tosylate, a borate (like tetrafluoroborate) ... In some cases, the salt can be not clearly ionized, such as for example silicate organic salts like tetraethyl orthosilicate (TEOS), trimethyl orthosilicate (TMOS), ...
An halide is preferably a chloride, a fluoride, a bromide or an iodide.
The oxidized metal is not necessarily soluble in water. For example, hexafluorophosphate salts or tetrafluoroborate salts can be suitable in organic solvents.

A carboxylate can be any suitable carboxylate ion, derived from a carboxylic acid, preferably a C₁-C₂₀, still preferably a C₁-C₁₀, branched or unbranched, substituted or unsubstituted, carboxylic acid and more preferably, the carboxylate is formate, acetate, propionate, butyrate, lactate, oxalate, citrate, trifluoroacetate or oxalate.

A metal can be any of the metals as defined in the periodic table of the elements, an in particular of the subclasses alkali metals, alkaline earth metals, lanthanides, actinides, transition metals, post transition metals, or metalloids. Preferably, the metal is a transition metal or a post transition metal. Preferably, the metal is selected from the list comprising silver, palladium, gold, platinum, copper, nickel, zinc, cadmium, indium, lead, aluminum, titanium, silicon, tantalum or iron.

A metal oxide is any oxide of the metals as defined above. Preferably, a metal oxide is a transition metal oxide, such as, but not limited to, titane oxide, tantalum oxide, iron oxide, copper oxide, silver oxide, nickel oxide or a post transition metal oxide, such as, but not limited to, silicon oxide, zinc oxide, cadmium oxide, indium oxide, lead oxide or aluminum oxide.

An alloy or mixture of metals is a combination of two or more metals and/or metal oxides as defined above. In some cases, an alloy comprises at least two metals and/or metal oxides from the same subclasses or from different subclasses. Preferably, an alloy comprises at least two metals or metal oxides from the subclass transition metal and/or post transition metal. Preferably, the alloy comprises at least two metals and/or metal oxides selected from the list comprising silver, palladium, gold, platinum, copper, nickel, zinc, cadmium, indium, lead, aluminum, iron, titanium, silicon, tantalum, and their respective oxides.
A metal base salt is for example silver nitrate, palladium dichloride, platinum dichloride, chloroauric acid, copper (II) acetate, iron (II) chloride, iron (III) chloride.

A nanomaterial according to the present invention is a particulate material (nanoparticle) having at least one dimension in the nanometric range, i.e. between 1 and 999 nm, preferably between 5 and 800 nm, still preferably between 10 and 500 nm, preferably below 250 nm or below 150 nm or below 100 nm as generally accepted for a nanomaterial.
A nanomaterial according to the present invention can have any shape, like for example, but not limited to a spherical shape, a cubical shape, a star shape, a rod shape, a wire shape, a nanocage or a triangular shape. The final shape of the nanomaterial is dependent on the nature of the metal-based element(s), the reacting conditions like pH, nature of the reducing agent, temperature, addition rate of the reducing agent, ...

The metal-based nanometarial core can be designed to have various properties by including one or more elements conferring for example optical properties (with e.g. gold, silver, copper, platinum), magnetic properties (with e.g. iron oxide), catalytic properties (with e.g. copper, palladium, titanium, platinum, tantalum) or antimicrobial properties (with e.g. silver or copper).

Calix[n]arenes are organic macrocycles wherein four or more phenolic structures are linked so as to form a crown, the linker between the phenolic structures usually consisting of CH₂, S, SO or SO₂. n designs the number of phenolic structures comprised in the macrocycle. n is typically comprised between 4 and 6.

When present on a nanomaterial surface, each of the aromatic subunits of the calixarene can adopt either an "up" or a "down" orientation towards the grafted surface. "Up" orientation refers to the phenolic groups pointing in the direction of the surface and "down" orientation refers to the phenolic groups pointing in the opposite direction, away from the surface.

A calix[n]arene diazonium salt designs a calix[n]arene wherein at least one phenolic ring is functionalized with a diazonium salt, as represented in formula I.

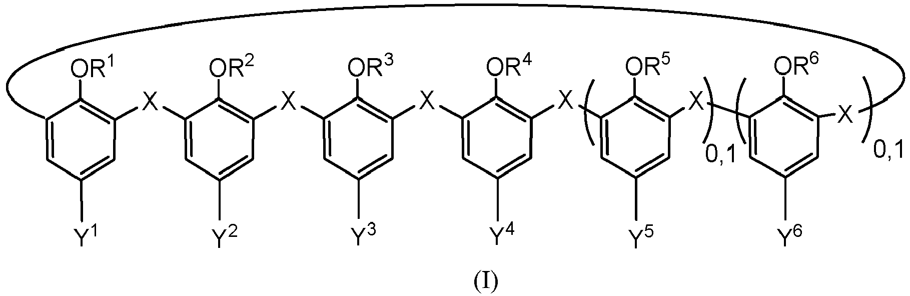

In one embodiment:
X represents CH₂, S, SO or SU₂; when X is S, the calixarene can be called a thiacalixarene;
R¹, R², R³, R⁴, R⁵ and R⁶ each independently represent hydrogen or C₁₋₃₀ alkyl optionally substituted with one or more substituents each independently selected from the group consisting of halo (e.g. fluoro, chloro, bromo, iodo), carboxylic acid ester, alkyl or benzyl thioester, alkenyl, alkynyl, C₁₋₃₀ alkoxy, aryl, substituted aryl (wherein the substituent is fluoro or cyano or C₁₋₃₀ alkyl or C₁₋₃₀ alkoxy), -N₃, cyano, carboxylic acid, carboxylic acid amide, -OH, amino, amido, imino, carbamate, acyl chloride, ureido, thioureido, mercapto, substituted disulfide, maleimide, heterocyclic, amino acid and amino acid derivative, peptide, protein, DNA, RNA, microRNA, phosphine or phosphine oxide, crown ether, aza-crown ether, cryptand, porphyrin, calixarene, cyclodextrin, resorcinarene, saccharide, and polyethylene glycol; and wherein two or more of R¹, R², R³, R⁴, R⁵ and R⁶ may be covalently linked either directly or by a bridge that includes oxygen, phosphine, phosphine oxide, sulfur, SO, SO₂, amino, imino, amido, ureido, thioureido, ester, thioester, alkene, alkyne or alkyl;
Y²-, Y³-, Y⁴- Y⁵- and Y⁶- are each independently selected from the group consisting of hydrogen, diazonium salt, OH, NO₂, halogen, C₁₋₃₀ alkyl, acyl, carboxylic acid and derivatives (e.g. ester, amide), -N₃ alkenyl or alkynyl;
Y¹- is a diazonium salt N₂⁺X⁻ where X⁻ represents an anion such as but not limited to BF₄⁻, PF₆⁻, Cl⁻, TsO⁻ (tosylate).

The index "0,1" at the right side of the aryl moieties bearing R⁵ and R⁶ in formula I means "0 or 1", meaning that these aryl moieties, each independently, can be present or absent.

The synthesis of calix[n]arene- diazonium salts can be performed either by in situ diazotation of the amino groups or by diazotation followed by isolation of the diazonium salts of formula I, according to procedures known in the art, as for example disclosed in EP2836539.

The calix[n]arenes bound to the surface of the metal-based nanomaterial is at least one of the compounds of formula (II) wherein: 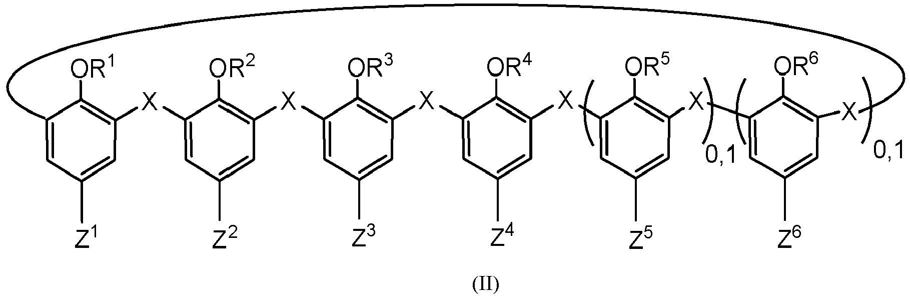
X represents CH₂, S, SO or SO₂;
R¹, R², R³, R⁴, R⁵ and R⁶ each independently represent hydrogen or C₁₋₃₀ alkyl optionally substituted with one or more substituents each independently selected from the group consisting of halo (e.g. fluoro, chloro, bromo, iodo), carboxylic acid ester, alkyl or benzyl thioester, alkenyl, alkynyl, C₁₋₃₀ alkoxy, aryl, substituted aryl (wherein the substituent is fluoro or cyano or C₁₋₃₀ alkyl or C C₁₋₃₀ alkoxy), -N₃, cyano, carboxylic acid, carboxylic acid amide, -OH, amino, amido, imino, carbamate, acyl chloride, ureido, thioureido, mercapto, substituted disulfide, maleimide, heterocyclic, amino acid and amino acid derivative, peptide, protein, DNA, RNA, microRNA, phosphine or phosphine oxide, crown ether, aza-crown ether, cryptand, porphyrin, calixarene, cyclodextrin, resorcinarene, saccharide, and polyethylene glycol; and wherein two or more of R¹, R², R³, R⁴, R⁵ and R⁶ may be covalently linked either directly or by a bridge that includes oxygen, phosphine, phosphine oxide, sulfur, SO, SO₂, amino, imino, amido, ureido, thioureido, ester, thioester,alkene, alkyne or alkyl;
Z²-, Z³-, Z⁴-, Z⁵- and Z⁶- are each independently selected from the group consisting of a covalent bond as defined for Z¹, hydrogen, diazonium salt, OH, NO₂, halogen, C₁₋₃₀ alkyl, acyl, carboxylic acid and derivatives (e.g. ester, amide), -N₃ alkenyl or alkynyl;
Z¹- is a covalent bond with the metal of the nanomaterial surface, said bond being a direct or indirect covalent bond.
A direct covalent bond, between the aromatic ring and the metal of the nanomaterial core may be obtained by loss of N₂ during the synthesis process. An indirect covalent bond may be obtained when N₂ is not lost during the synthesis process and remains as a linker between the aromatic ring and the metal.
The formation of a direct or indirect covalent bond may depend on the nature of the metal, the nature of the calixarene and/or the synthetic conditions.

It will be appreciated that groups having similar references in formula (I) and formula (II) are indeed similar, the nanomaterial of formula (II) resulting from the reaction of at least one of the diazonium salts of formula (I) according to the method of the invention. The following part of the present description will therefore refer to both compounds of formula (I) and compounds of formula (II). Chemical groups differing between the diazonium salt of formula (I) and the nanomaterial of formula (II) are noted differently.

Also preferably, at least one of Y²-, Y³-, Y⁴- Y⁵- and Y⁶- (in addition to Y¹-) is a diazonium salt.

Conversely, at least one of Z²-, Z³-, Z⁴- Z⁵- and Z⁶ is a bond with the metal of the nanomaterial surface.

Possibly, the calix[n]arene of fomula I bears one, two, three, four or, if applicable, five or six diazonium groups, and an equal number of bonds are formed with the surface of the material. This may be applicable for the less flexible calix[n]arenes, in particular where n is 4, or with appropriately substituted calix[n]arenes. This may also be applicable for the more flexible calix[n]arenes, such as the calix[5]arenes or the calix[6]arenes which can be modified by adding appropriate substituents on the small rim or covalent bridges between the phenolic moieties (i.e. where two or more of R¹, R², R³, R⁴, R⁵ and R⁶ are covalently linked either directly or through a bridge as defined above).

The skilled person will be able to select the number of bonds to the surface per calixarene moiety based on the conformational flexibility of the (thia)calix[n]arene moiety, or on the possibilities in terms of chemical configuration, in particular as regards steric hindrance.

Preferably, the calix[n]arene is a calix[4]arene.

Preferably, the calix[n]arene is a calix[4]arene wherein at least one of Y²-, Y³- and Y⁴- is a diazonium salt, preferably at least two of Y²-, Y³- and Y⁴- are diazonium salts, preferably Y²-, Y³- and Y⁴- are diazonium salts.

Preferably, R¹, R², R³, R⁴, R⁵ and R⁶ each independently represent a C₁₋₃₀ alkyl optionally substituted with one or more R substituents each independently selected from the group consisting of C₁₋₃₀ alkoxy, -N₃, cyano, carboxylic acid, carboxylic acid amide, acid halide, amide ester, -OH, amino, amido, imino, carbamate, maleimide, thiol, cyanate, isocyanate or acyl chloride.

Preferably, R¹, R², R³, R⁴, R⁵ and R⁶ each independently represent polysaccharides, CH₂COOH, CH₂(CH₂OCH₂)ₘCH₂OCH₃, CH₂(CH₂OCH₂)ₚCH₂OCH₂COOH or CH₂(CH₂OCH₂)_{q}CH₂R, wherein R is as defined above , wherein m, p and q are not limited but are, each independently, preferably 50 or below.

The metal-based nanomaterial MC of the invention is coated with calix[n]arenes, meaning that a particulate nanomaterial is formed with a metallic central core M having calix[n]arenes bound to its surface so as to form an ultrathin layer C, as illustrated in Formula III.

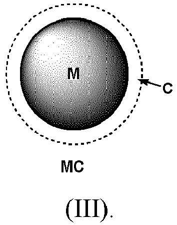

The ultrathin layer of calix[n]arenes is preferably a monolayer, meaning that the thickness of the ultrathin monolayer is the thickness of a calix[n]arene, including its substituents and functional groups.

Combinations of metal-based salts can be used, leading to the formation of mixed metal-based central cores, i.e. metal alloys.

Combinations of calix[n]arenes-diazonium salts can be used, leading to the formation of mixed layers whose composition can be controlled. As calix[n]arenes can be further functionalized, depending on the nature of R¹, R², R³, R⁴, R⁵ and R⁶, this presents the advantage of offering the possibility to confer a plurality of functionalities to the metal-based nanomaterials coated with calix[n]arenes.

### Detailed Description of the Invention

Figure 1: UV-visible spectrum of Ag-C1-20 at various pH;
Figure 2: UV-visible spectrum of Ag-C1-20 in presence of KF over time;
Figure 3: UV-visible spectrum of Ag-citrate (left) and Ag-S-PEG (right) at pH 7 and in presence of KF;
Figure 4: IR spectrum of Ag-C1-20;
Figure 5: TEM images of Ag-C1-20;
Figure 6: UV-visible spectrum of particles obtained in example 2a, 2b, 2c;
Figure 7: TEM images of particles obtained in example 2a, 2b, 2c;
Figure 8: IR spectrum of Ag-C1 post functionalized;
Figure 9: E. Coli growth inhibition study by disc diffusion method for different conditions (1= water; 2= Ag-citrate; 3= Ag-C1; 4= Ag-C2; 5= 60 µM Kanamycine; 6= 60 mM Kanamycine) and right: optical density at 600 nm of a suspension of bacteria after 12 hours in different conditions (same numbering of the samples as for the disc diffusion method);
Figure 10: UV-visible spectrum of Ag-C2 at various pH (left) and in presence of KF over time (right);
Figure 11: IR spectrum of Ag-C2;
Figure 12: TEM images of Ag-C2;
Figure 13: UV-visible spectrum of Ag-C3 at various pH (left) and in presence of KF over time (right);
Figure 14: IR spectrum of Ag-C3;
Figure 15: TEM images of Ag-C3;
Figure 16: UV-visible spectrum of Ag-C1C2, for various ratio of C1 to C2, at various pH;
Figure 17: UV-visible spectrum of Ag-C1C2, for a ratio of C1 to C2 of 50:50, in presence of KF over time;
Figure 18: IR spectrum of Ag-C1 C2, for various ratio of C1 to C2, overlaid;
Figure 19: UV-visible spectrum of Ag-C1C3, for various ratio of C1 to C3, at various pH, and graph displaying the shift of absorption peak in function of ratio of C3;
Figure 20: UV-visible spectrum of Ag-C1C3, for a ratio of 85% C3, in presence of KF over time;
Figure 21: IR spectrum of Ag-C1C3, for various ratio of C1 to C3, overlaid;
Figure 22: UV-visible spectrum of Ag-C2C3, for various ratio of C2 to C3, at various pH, UV-visible spectrum of Ag-C2C3, for a ratio of 50% C3, in presence of KF over time;
Figure 23: IR spectrum of Ag-C2C3, for various ratio of C2 to C3, overlaid;
Figure 24: UV spectrum of Au-C1 at various pH, and in presence of KF over time, compared with spectrum in the same conditions for Au-citrate and Au-MUA;
Figure 25: IR spectrum of Au-C1, before and after functionalization with NH2-PEG7-OCH3 (Au-C1-A) and peptide of sequence AAPLSQETFSDLWKLL (Au-C1-B);

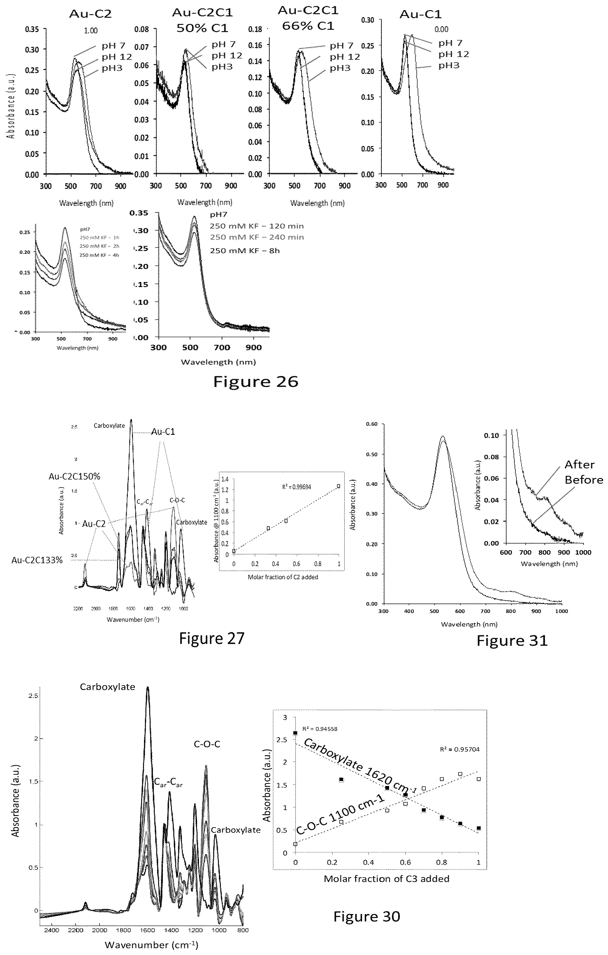

Figure 26: UV-visible spectrum of Au-C2C1, for various ratio of C2 to C1, at various pH, UV-visible spectrum of Au-C2C1, for ratios of 0% and 50% C1, in presence of KF over time;
Figure 27: IR spectrum of Au-C1C2, for various ratio of C1 to C2, overlaid;

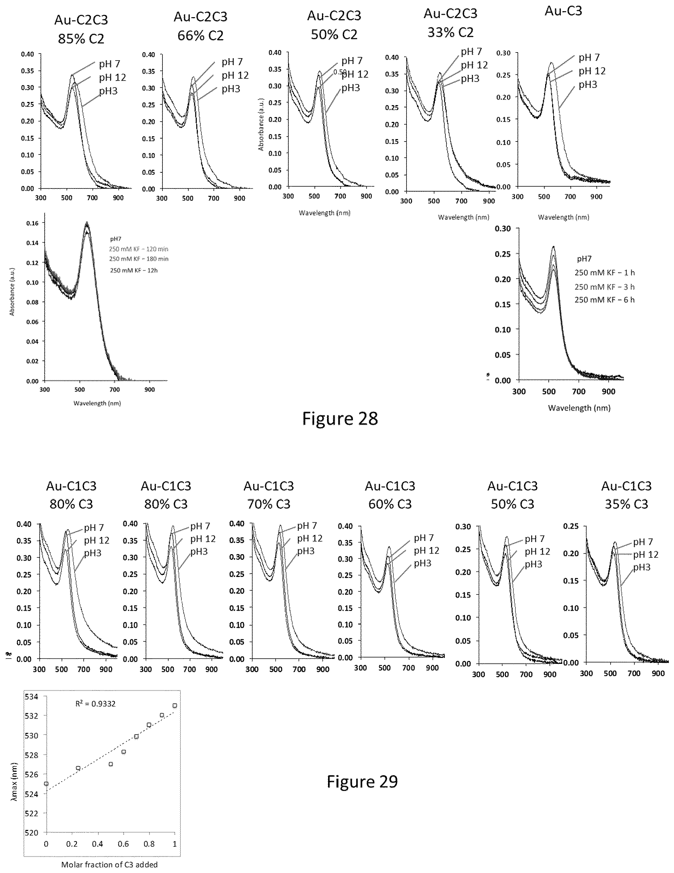

Figure 28: UV-visible spectrum of Au-C2C3, for various ratio of C2 to C3, at various pH, UV-visible spectrum of Au-C2C3, for ratios of 0% and 85% C2, in presence of KF over time;
Figure 29: UV-visible spectrum of Au-C1C3, for various ratio of C1 to C3, at various pH, and graph displaying the shift of absorption peak in function of ratio of C3;
Figure 30: IR spectrum of Au-C1C3, for various ratio of C1 to C3, overlaid, and graph displaying the evolution of selected peaks intensity in function of ratio of C3;
Figure 31: IR spectrum of Au-C1C3 with 60% C3, before and after functionalization with Cyanine dye with terminal amino group absorbing at 800 nm;

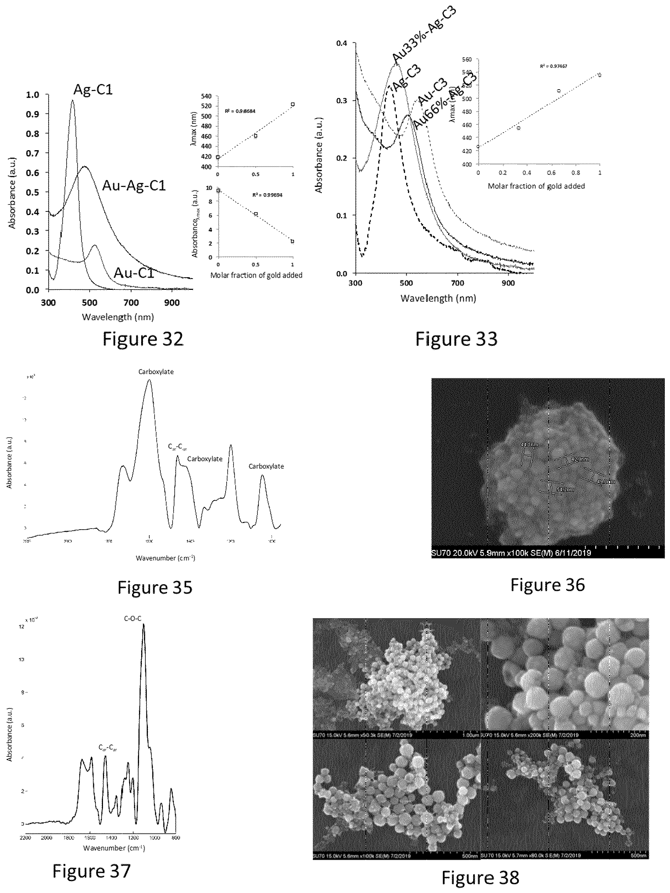

Figure 32: UV spectra of Ag-C1, Au-C1 and AuAg-C1;
Figure 33: UV spectra of Ag-C3, Au-C3 and AuAg-C3 (33% Au and 66% Au);
Figure 34: overlaid IR spectrum of Ag-C3, Au-C3 and AuAg-C3 (33% Au and 66% Au);
Figure 35: IR spectrum of Pd-C1;
Figure 36: SEM image of Pd-C1;
Figure 37: Figure 35: IR spectrum of Pd-C3;
Figure 38: TEM image of Pd-C3;

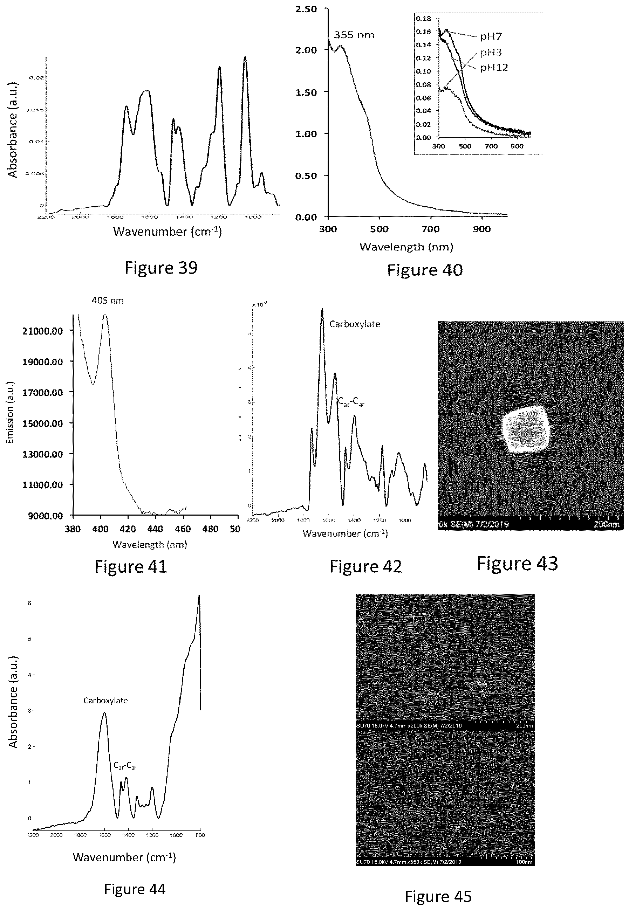

Figure 39: IR spectrum of Pt-C1;
Figure 40: UV spectrum of Cu-C1 at various pH;
Figure 41: florescence emission spectrum of Cu-C1 demonstrating presence of copper oxide particles;
Figure 42; IR spectrum of Cu-C1;
Figure 43: SEM image of Cu-C1;
Figure 44: IR spectrum of Fe-C1
Figure 45: SEM images of Fe-C1;

The term "about" when used in relation to a numerical value has the meaning generally known in the relevant art. In certain embodiments the term "about" may be left out or it may be interpreted to mean the numerical value +10%; or +5%; or +2%; or +1%.

The term "thickness" refers to the distance between the surface that is grafted and the part of the grafted (thia)calix[n]arene molecule that is furthest away from the surface. Usually an ultrathin layer of calixarene is grafted onto the metallic core. The ultrathin layer is preferably a monolayer. An ultrathin layer has a thickness of about 1 nm to 15 nm, corresponding to the calixarene rings without any further substituents (i.e. not counting R¹, R², R³, R⁴, R⁵ and R⁶ groups)

The terms "rather dense" and "dense" are used to describe a surface of a material that is coated with molecules in such way that molecules considered as single spheres or cylinders occupy an area equivalent to more than 50%, or more than 60 %, or more than 70%, of a close-packed organization of the spheres or cylinders according to the compact van der Waals model.

The term "alkyl" refers to non-aromatic hydrocarbon groups. In particular "alkyl" refers to linear or branched, cyclic (e.g. cycloalkyl) and non-cyclic (acyclic) hydrocarbon groups. These may be unsaturated (see "alkenyl" and "alkynyl" below) or saturated. They can have varying numbers of carbon atoms, e.g. up to about 30, or up to about 20, or up to about 15, or up to about 10 carbon atoms. Alkyl groups thus include C₁₋₃₀ alkyl, C₁₋₁₀ alkyl (as more specifically defined below), C₁₋₆ alkyl, and C₁₋₄ alkyl groups.

The term "C₁₋₁₀ alkyl" denotes straight and branched saturated hydrocarbon radicals having from one to ten carbon atoms such as, for example, methyl, ethyl, 1-propyl, 2-propyl, 1-butyl, 2-butyl, 2-methylpropyl, 1-pentyl, 2-pentyl, 2-methylpropyl, 1-hexyl and other hexyl isomers, 1-heptyl and other heptyl isomers, 1-octyl and other octyl isomers, 1-nonyl and other nonyl isomers, 1-decyl and other decyl isomers. The term "C₁₋₆ alkyl" include one to six carbon atoms. "C₁₋₄alkyl" have from one to four carbon atoms. Of particular interest are straight (non-branched) C₁₋₁₀alkyl, C 1-6 alkyl, or C 1-4 alkyl groups.

In the embodiments where two or more of R¹, R², R³, R⁴, R⁵ and R⁶ form a bridge (bridging group) selected from phosphine, phosphine oxide, amino, imino, amido, ureido, thioureido, ester, thioester, alkene, alkyne or alkyl, one of the hydrogen atoms or substituents on these moieties are replaced by a bond.

The calix[n]arene-based diazonium salts of formula I can be prepared by reacting a calix[n]arene bearing amino NH₂ groups with a nitrite such as sodium nitrite, in an aqueous acidic solution or with an alkyl nitrite such as isoamylnitrite or tertio-butylnitrite in an organic solvent (e.g. dichloromethane, polar aprotic solvents such as acetonitrile, dimethylformamide, dimethylacetamide, and the like solvents) or with nitroso salt (NOX) in organic solvents (e.g. acetonitrile). The diazonium salts of formula I, preferably the BF₄⁻ salts, can be prepared from an ice-cold solution of the corresponding amino NH₂ derivatives in HBF₄ by the slow addition of NaNO₂ (in excess) dissolved in a minimum amount of water. The precipitate is filtered off, washed with H₂O. The diazonium salts of formula I, preferably its BF₄⁻ salts, can be prepared from a solution of the corresponding amino NH₂ derivatives in acetonitrile in the presence of NOBF₄ (preferably in a slight molar excess) at low temperature (e.g. -40°C). Typically, the crude residue is then washed with diethylether and/or ethanol.

The ultrathin layer of grafted (thia)calix[n]arenes is a homogeneous layer and does not present the large ramifications typically encountered with other systems, which lead to a complex and irregular surface coating. The grafted (thia)calix[n]arenes may form a rather dense coating, so that little free surface of the coated material is present.

Classical (bio)conjugation reactions such as peptide type coupling, maleimide-thiol reaction or copper catalyzed Huisgen cycloaddition (click chemistry) can be used for the immobilization of molecules or biomolecules on the grafted (thia)calix[n]arene ultrathin layer, the choice of the reaction depending on the groups present on the grafted (thia)calix[n]arenes. As a representative example, the grafted (thia)calix[n]arene ultrathin layer, when functionalized with a carboxylic acid group, can be esterified or converted with an appropriate amine into amides. Appropriate amines include not only simple amines but also amino acids, peptides, proteins DNA, RNA, microRNA, and various chemical species (such as ligands for metal ions or for anions, molecular receptors, oligomers or polymers) with one or multiple appending amino arms. The carboxylic acid group can further be linked to hydroxyl-containing species such as saccharides, cyclodextrins and polyethylene glycols.

The covalent surface grafting of functionalized (thia)calix[n]arenes (with e.g. COOH, maleimide or alkyne groups on the small rim) on the large rim provides well-organized and compact monolayers, which can be post-functionalized. In other words, grafted (thia)calix[n]arenes induce a pre-structuration and a pre-functionalization of the surface at the molecular level.

The grafted materials of the invention can be used as a versatile platform for further modification, in particular the anchoring of further molecules resulting a regular and possibly rather dense molecular layer of various chemical species (molecules, nanoparticles, biomolecules, ligands for metal ions or anions, molecular receptors, oligomers or polymers, etc.) on conducting or semiconducting or non-conducting material surfaces. The method of the invention enables to control the density of functional groups present at the surface of the nanomaterials in order to introduce post-functionalization.

The calixarene layer can comprise various calixarenes.

The examples below make use of three types of calixarenes: a functional calixarene (Cl), a functional PEGylated calixarene (C2) and a PEGylated calixarene (C3). The versatility of this procedure is even greater as the core can be made of metal alloys and the organic layer can be a mixed layer of calixarenes in order to combine their different properties.

### General synthetic scheme

Scheme 1 illustrates the general method of the invention to convert a mixture of at least one metal-based salt with at least one calix[4]arene diazonium salt (C1, C2 and/or C3) to obtain a metal-based nanomaterial with a core being pure metal or an alloy, coated with a calixarene layer, of either single type or combined type.

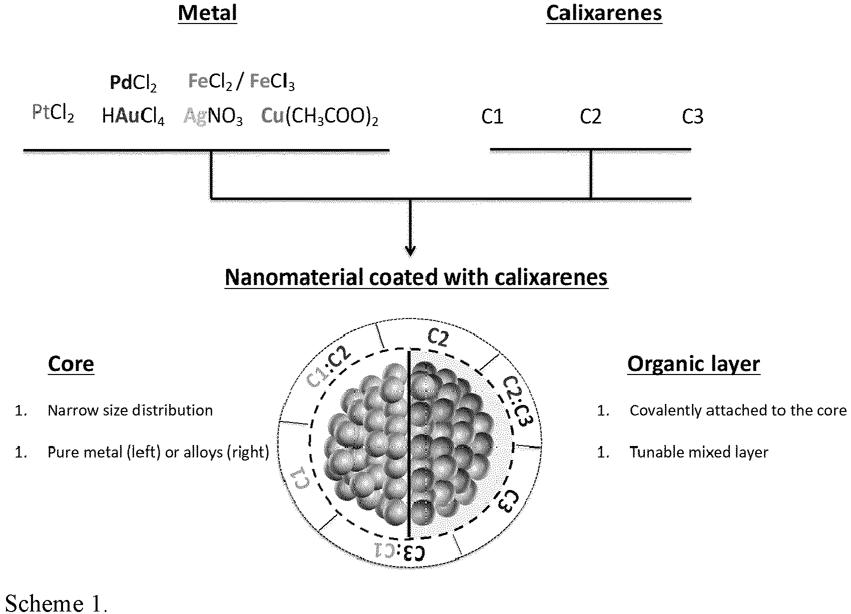

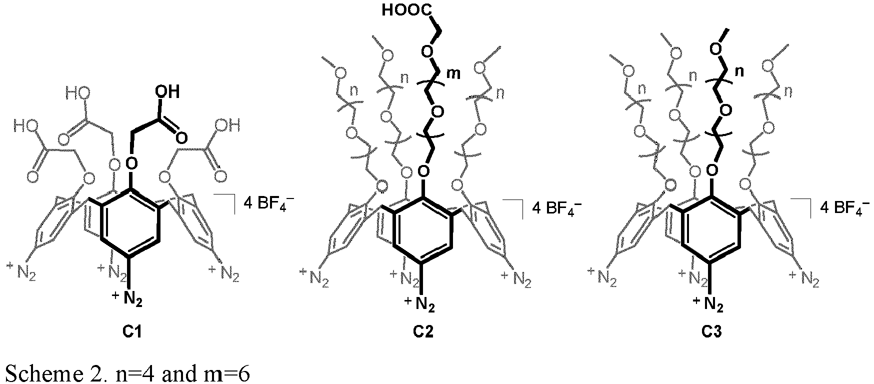

Scheme 2 shows the three calix[4]arenes that were used to make different layers, each possessing unique properties:
- C1: this calixarene is negatively charged at pH values above 6, which ensures the stability of the colloids thanks to electrostatic repulsion between the particles. The carboxyl groups also allow post-functionalization of the particles with an amine or hydroxy-containing (bio)molecule via the formation of an amide or ester group using common coupling procedures. The particles coated with C1 are pH sensitive and precipitate at low pH. This aggregation should however be reversible and the particles easily resuspended in basic conditions.
- C2: this calixarene bears three PolyEthylene Glycol (PEG) chains ended by a methoxy group and one terminated by a carboxylic acid. This calixarene displays one protonable/deprotonable group, which should lead to particles that are only weakly pH sensitive. Furthermore, PEG is considered as the gold standard polymer to ensure biocompatibility and antifouling properties to nanomaterials. The presence of one carboxylic group allows post-functionalization of the particles.
- C3: this calixarene bears four Polyethylene Glycol (PEG) chains ended by a methoxy group, which should lead to pH insensitive colloids.

Mixed layers of these calix[4]arenes possess combination of these properties with the possibility to provide to the functionalized particles the desired properties by controlling the proportion of the calix[4]arenes within the mixed layer.

### General synthesis procedure 1.

In a protein LoBind eppendorf, 150 µL of a 10 mM metal salt solution are mixed successively with 575 µL of milliQ water and 360 µL of a solution of a 5 mM calixarene milliQ water and the pH is adjusted to 6.5 using a NaOH solution (1M). Quickly after this, 410 µL of a 15 mM sodium ascorbate solution are added and the Eppendorf is placed overnight in a thermomixer at 60 °C and agitated at 800 RPM.

The sodium ascorbate is present at around 4 mM concentration in the reaction mixture.

After 16 hours, the nanoparticles are centrifuged (20 minutes at 18.000g) and the supernatant is removed and replaced by around 1500 µL of a washing medium. This washing procedure is repeated four times. The medium replacing the supernatant depends on the calixarene used (table 1). In the case of silver nanoparticles , the final concentration of nanoparticles obtained after the washing step is evaluated by measuring the extinction coefficient of the silver particles following the method disclosed in D. Paramelle, A. Sadovoy, S. Gorelik, P. Free, J. Hobley and D. G. Fernig, A rapid method to estimate the concentration of citrate capped silver nanoparticles from UV-visible light spectra, Analyst, 2014 139, 4855*.*

**Table 1: medium used to replace the removed supernatant during the cleaning steps of nanoparticles.**

| | C1 | C2 | C3 | C1:C2 | C1:C3 | C2:C3 |
|---|---|---|---|---|---|---|
| 1^{st} cycle | Water* | SDS** | SDS** | SDS** | SDS** | SDS** |
| 2^{nd} cycle | Water* | SDS** | SDS** | SDS** | SDS** | SDS** |
| 3^{rd} cycle | Water* | Water* | Water* | Water* | Water* | Water* |
| 4^{th} cycle | Water* | Water* | Water* | Water* | Water* | Water* |

| | | | | | | |
|---|---|---|---|---|---|---|
| * Lichrosolv water; **1% SDS solution in weight | | | | | | |

When the refractive index of a particle is known, the final concentration of particles can also be measured by DLS measurement (Malvern-Zetasizer) to give the concentration of particles (particles/mL).

Alternatively, the number of produced particles can be extrapolated from the quantity of reduced metal (the difference between the total quantity of metal added and the quantity of remaining cations in the solution, measured for example by ICP), the average size of the particles (obtained by TEM) and the density of the metal.

The UV-Visible spectrum of the coated nanomaterial is recorded using an absorption spectrometer. The coated nanomaterials are typically diluted 10 to 12 times in pure water for recording the UV-Visible spectra.

The colloidal stability is evaluated by comparing UV-Vis spectrum of the coated nanomaterial at pH 3, pH 7 and pH 12. Alteration of the UV-Visible spectrum at pH 3 is the sign of particles aggregation. Reversibility can be evaluated by assessing recovery of the UV-Vis spectrum when increasing the pH.

The chemical stability of the coated nanomaterial is evaluated by recording the UV-Visible spectra of the coated nanomaterial in solution at different time intervals after addition of a 150 mM potassium fluoride solution. No or little alteration of the UV-Visible spectrum means that the colloidal stability is not affected by the addition of KF, indicating that the metallic surface is not accessible to the fluoride ions, which is a sign of a dense and homogeneous repartition of the calixarenes on the surface of the metal-based core.

The presence of calixarenes on the metallic core is confirmed by IR spectroscopy.

The size and the shape of the metallic core of the particles was determined by analysis with SEM (scanning electron microscopy) and/or TEM (transmission electron microscopy).

The hydrodynamic diameter of the particles is analyzed by Dynamic Light Scattering (DLS) at a 100x dilution in water.

General observations for the experimental conditions:
- a pH around 6.5 (±1) of the reaction medium leads to the best coverage of nanoparticles with calixarenes.
- the core size can be tuned by changing the reductive strength of the medium in which the particles are synthesized. The nature and amount of the reducing agent in the general synthesis procedure (sodium ascorbate) leads to the formation of approximately 20 nm particles, which is the most commonly desired size for biomedical applications.

### Example 1: silver core nanomaterials coated with calix[n]arenes of type C1: Ag-C1-20

Nanoparticles with pure silver core and coated with calixarene C1 were synthesized according to the general synthesis procedure 1, using silver nitrate as silver salt.

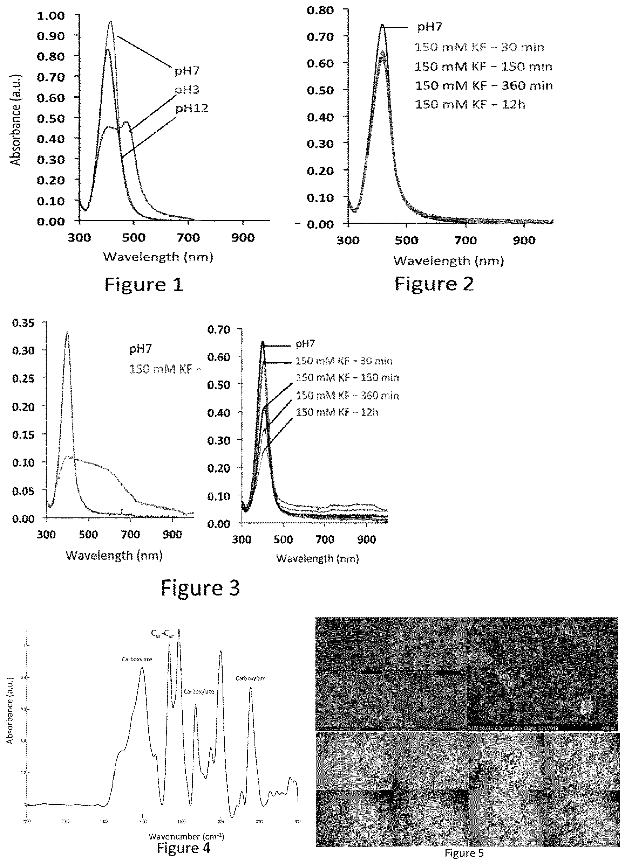

The **UV-visible spectrum** of Ag-C1-20 (Figure 1) shows a sharp absorption peak at 418 nm, revealing well-dispersed silver nanoparticles with a narrow size distribution.

The final concentration of nanoparticles was estimated to be around 2.5 nM, based on a calculated extinction coefficient of 4.10⁹ L.mol⁻¹.cm⁻¹, which is in good agreement with the extinction coefficient reported in the literature for particles of that size (20 nm as shown by TEM).

**Colloidal stability** is affected at low pH the Ag-C1-20 particles are aggregated as the carboxylic acids of C1 are protonated, increasing the initially negative surface potential of the particles, and decreasing the electrostatic repulsion between them. A typical spectrum of DLCA (Diffusion Limited Colloidal Aggregation) aggregated particles with a second absorption band around 490 nm is observed. However, this aggregation is reversible by increasing the pH thanks to the highly protective calixarene layer.

**Chemical stability** - After 12 hours of exposure to potassium fluoride, only few particles were degraded, meaning that the silver surface is hardly accessible to the fluoride thanks to the dense and strongly anchored calixarene layer (Figure 2).

**The IR spectrum** of Ag-C1-20 (figure 4) clearly shows absorption peaks of the aromatics (1458 cm-1) and of the carboxylates (1620, 1350, 1050 cm-1) of the calixarene.

SEM and TEM showed spherical particles with a core size of around 20 nm (Figure 5).

The **hydrodynamic diameter** of Ag-C1-20 diluted 100 times in milliQ water at 25 °C is around 35 nm

### Comparative example 1:

The chemical stability of commercially available silver nanoparticles, coated with citrate (Ag-Citrate, S-20-XX, Cytodiagnostics (Burlington, Canada)) or by a thiolated-PEG (Ag-SPEG, SC3K-20-X*, Cytodiagnostics (Burlington, Canada)) was recorded for comparison under the same conditions as Ag-C1.

As shown on figure 3, in presence of KF, Ag-Citrate is almost completely degraded after 10 min, while Ag-SPEG slowly degrades overtime, revealing the robustness of the calixarene coating.

### Example 2a, 2b, 2c: size variation of silver core nanomaterials coated with calix[n]arenes of type C1

Nanoparticles with pure silver core and coated with calixarene C1 were synthesized according to the general synthesis procedure 1 using silver nitrate as silver salt, but using, instead of a final 4 mM sodium ascorbate concentration:
a) aqueous sodium borohydride (NaBH₄), at 4 mM, which is a stronger reducing agent than ascorbate
b) a sodium ascorbate at 4 mM, as in example 1,
c) sodium ascorbate at 1 mM.

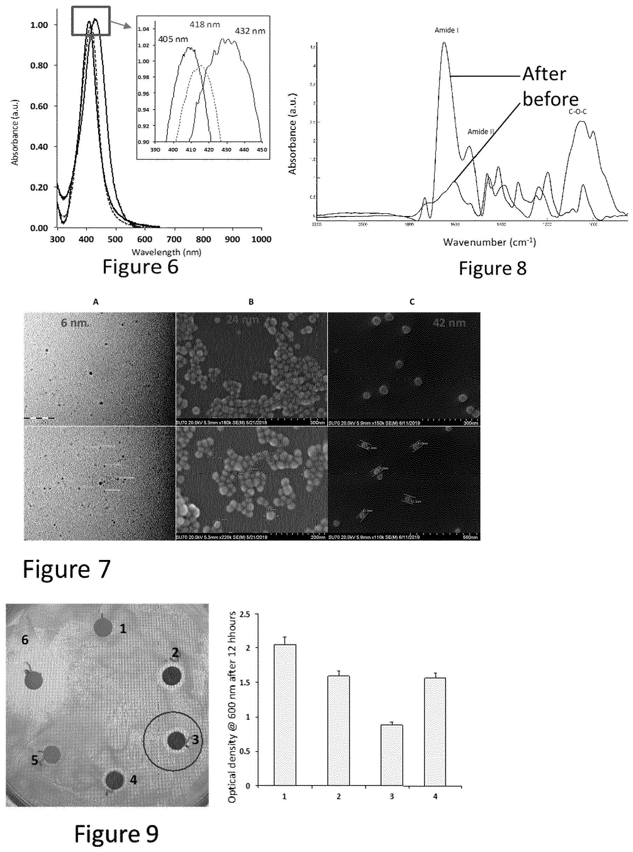

The UV-Visible spectra of the resulting nanoparticle suspensions are shown in figure 6. TEM pictures are shown in figure 7.
Example 2a: the particles show an absorbance peak at 405 nm, indicating smaller particles were synthesized compared to example 1. This is confirmed by TEM analysis that reveals a core size of 6 nm for these particles.
Example 2b: the particles show an absorbance peak at 418 nm, as in example 1. This is confirmed by TEM analysis that reveals a core size of 24 nm for these particles.

Example 2c: the particles show an absorbance peak at 432 nm, indicating that larger particles were synthesized compared to example 1. This is confirmed by TEM analysis that reveals a core size of 42 nm for these particles.

### Example 3: functionalization of Ag-Cl

Calixarene C1 bears functional carboxylate groups, allowing additional chemical reactions (post-functionalization of the surface). Formation of an amide bond between carboxylates of Ag-C1 and ligands containing an amine group was demonstrated with NH₂-PEG₇-OCH₃ (Figure 8, left) and a peptide, whose sequence is AAPLSQETFSDLWKLL (Figure 8, right).

Figure 8 displays the IR spectra of calixarene coated nanomaterial Ag-C1 post-functionalized with NH₂-PEG₇-OCH₃ (Figure 8, left) or the above-mentioned peptide (Figure 8, right). Peaks corresponding to an amide bond are clearly present around 1650 cm⁻¹.

### Example 4: silver core nanomaterials coated with calix[n]arenes of type C2: Ag-C2

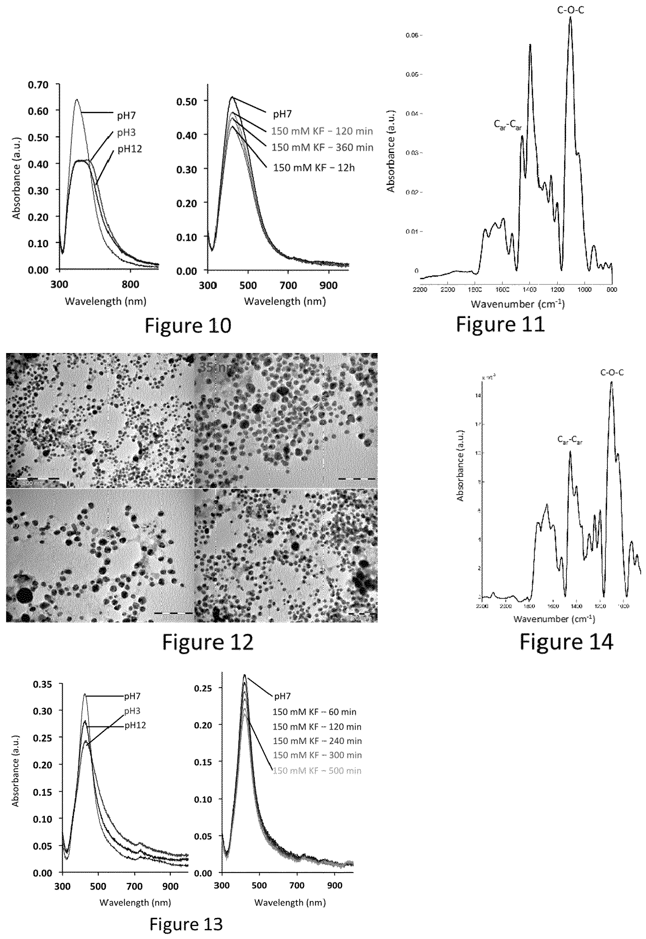

Nanoparticles with pure silver core and coated with calixarene C2 were synthesized according to the general synthesis procedure 1, using silver nitrate as silver salt.
The **UV-visible spectrum** of Ag-C2 (Figure 10) shows a sharp absorption peak at 424 nm, revealing well-dispersed silver nanoparticles with a narrow size distribution.
The final concentration of nanoparticles was estimated to be around 0.4 nM, based on a calculated extinction coefficient of 2.10¹⁰ L.mol⁻¹.cm⁻¹, which is in good agreement with the extinction coefficient reported in the literature for particles of that size (35 nm as shown by TEM).
**Colloidal stability and chemical stability** are affected by pH variation: during a pH variation cycle (neutral - acidic - basic) and upon KF exposure a loss of Ag-C2 particles is observed, both indicating a less efficient coating than in the case of Ag-C1 calixarenes (Figure 10, left). However, the resistance of these particles to fluoride etching (Figure 10, right) is still much higher than that of commercially available particles (ordered from Cytodiagnostics, Burlington, Ontario, Canada; core size of 20 nm; coated with a HS-PEG-OCH₃ (Mw = 3000 Da).

**The IR spectrum** of Ag-C2 (figure 11) clearly shows absorption peaks of the aromatics (1458 cm⁻¹) and of the polyethyleneglycol (PEG) chains (1100 cm⁻¹) of the calixarene.

SEM and TEM showed spherical particles with a core size of around 35 nm (Figure 12).

### Example 5: antimicrobial properties of Ag-C1-20 and Ag-C2

In addition to interesting optical properties, the interest of silver cores lies in their antimicrobial activity. Despite the dense and stable coating of Ag-C1, these particles express similar or even greater antimicrobial activity than citrate-capped silver nanoparticles (Ag-Citrate).

E. Coli growth inhibition was studied by disc diffusion method for different conditions: 1= water; 2= Ag-citrate; 3= Ag-C1; 4= Ag-C2; 5= 60 µM Kanamycine; 6= 60 mM Kanamycine).
Figure 9 shows on the left part the test plate after 12 h, and on the right part, the optical density at 600 nm of bacteria suspension in presence of 1-water, 2= Ag-citrate, 3=Ag-C1-20 and 4= Ag-C2, after 12 hours.
From the test plate, it is clear that Ag-C1 and Ag-C2 are as efficient as Ag-Citrate in the case of the disc diffusion method and more efficient than 60 µM of Kanamycine, even if this well-known antibiotic is more than 1000 times more concentrated than the particles ([AgNPs] = 0.6 nM and [Kanamycine] = 60 µM).
In the case of the inhibition in solution, a stronger growth inhibition is observed with Ag-C1 than with Ag-Citrate, characterized by a lower optical density at 600 nm, after a defined incubation time, which could be explained by a longer lifetime of the Ag-C1 in the culture medium due to the protective calixarene-based layer. Ag-C2 is as efficient as Ag-Citrate.

### Example 6: silver core nanomaterials coated with calix[n]arenes of type C3: Ag-C3

Nanoparticles with pure silver core and coated with calixarene C3 were synthesized according to the general synthesis procedure 1, using silver nitrate as silver salt.
The **UV-visible spectrum** of Ag-C3 (Figure 13) shows a sharp absorption peak at 432 nm, revealing well-dispersed silver nanoparticles with a narrow size distribution.
The final concentration of nanoparticles was estimated to be around 0.4 nM, based on a calculated extinction coefficient of 2.10¹⁰ L.mol⁻¹.cm⁻¹, which is in good agreement with the extinction coefficient reported in the literature for particles of that size (around 46 nm as shown by TEM).

**Colloidal stability and chemical stability** are only slightly affected by pH variations (Figure 13 left) and KF exposure (Figure 13 right). This is due to the presence of the uncharged PEG layer capping the particles, which provide steric stabilization of the colloidal suspension instead of the electrostatic stabilization (which is the case for C1).

**The IR spectrum** of Ag-C3 (figure 14) clearly shows absorption peaks of the aromatics (1458 cm⁻¹) and of the polyethyleneglycol (PEG) chains (1100 cm⁻¹) of the calixarene.

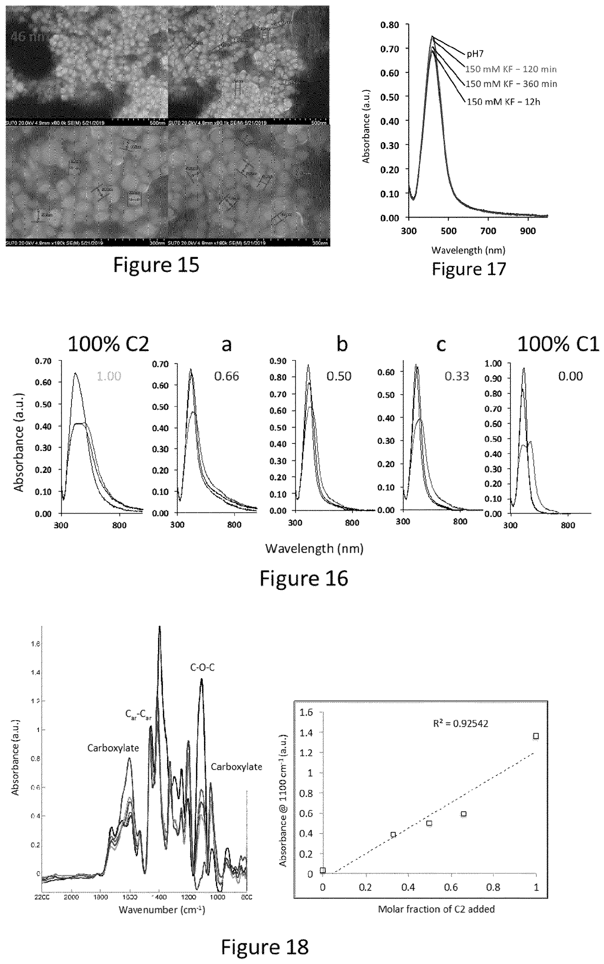

SEM and TEM showed spherical particles with a **core size** of around 46 nm (Figure 15).

### Example 7: silver core nanomaterials coated with calix[n]arenes of type C1 and C2: Ag-C1C2

Nanoparticles with pure silver core and coated with a mixture of calixarenes C1 and C2, in various ratio, were synthesized according to the general synthesis procedure 1.

The results are summarized in table 1 below.

**Table 1.**

| Molar fraction of C2 | 1 (Ag-C2) | 0.66 | 0.5 | 0.33 | 0 (Ag-C1-20) |
|---|---|---|---|---|---|
| UV absorption peak | 426 | | | | 418 |
| Colloidal stability | Idem example 4 - high resistance | Minor reversible aggregation | Minor reversible aggregation | Reversible aggregation | Idem example 1 - reversible aggregation |
| Chemical stability | | | No loss of particles | | |
| | | | | | |

The **UV-visible spectra** of the Ag-C1C2 (Figure 16) show sharp absorption peaks between 426 and 418 nm, revealing well-dispersed silver nanoparticles with a narrow size distribution.
The final nanoparticle concentrations were estimated to be around 0.4-2.5 nM, based on the calculated extinction coefficient.

**Colloidal stability and chemical stability:** The chemical robustness of these hybrid Ag-C1C2 particles is demonstrated by exposing the particles to extreme conditions, either acidic (figure 16) or etching environment (figure 17). After a pH variation cycle, we observe high resistance of the hybrid particles against acidic conditions, without loss of particles, unlike in the case of pure Ag-C2. A mixed layer of calixarene C1:C2 increases the chemical robustness of these particles.
Figure 17 shows the etching resistance for the Ag-C1C2 in a 1:1 ratio. These particles can endure fluoride exposure during several hours without significant loss.
**The IR spectrum** of Ag-C1C2 (figure 18) clearly shows absorption peaks of the aromatics (1458 cm⁻¹), the carboxylates (1620, 1350, 1050 cm⁻¹) and of the polyethyleneglycol (PEG) chains (1100 cm⁻¹) of the calixarene for all calixarene ratios, indicating that the calixarenes are all well grafted onto the surface. Furthermore, the intensity of the PEG signal depends on the amount of C2 added during the synthesis (the IR spectra have been normalized using the ring stretch band of the calixarenes at 1458 cm⁻¹), thereby confirming that it is possible to (i) synthesize particles covered with a mixed layer of calixarenes and (ii) control the proportions of calixarenes within the mixed layer.

### Example 8: silver core nanomaterials coated with calix[n]arenes of type C1 and C3: Ag-C1C3

Nanoparticles with pure silver core and coated with a mixture of calixarenes C1 and C3, in various ratio, were synthesized according to the general synthesis procedure 1.
The results are summarized in table 2 below.

**Table 2.**

| Molar fraction of C2 | 1 (Ag-C3) | 0.95 | 0.90 | 0.85 | 0.80 | 0.70 | 0.60 | 0.5 | 0.35 | 0 (Ag-C1-20) |
|---|---|---|---|---|---|---|---|---|---|---|
| UV peak (nm) | 432 | | | | | | | | | 418 |

| Colloidal stability | Idem example 6 - high resistance | High resistance to acidic conditions | High resistance to acidic conditions | High resistance to acidic conditions | High resistance to acidic conditions | Minor reversible aggregation | Minor reversible aggregation | Minor reversible aggregation | Reversible aggregation | Idem example 1 - reversible aggregation |
|---|---|---|---|---|---|---|---|---|---|---|
| Chemical stability | | | | No loss of particles | | | | | | |
| Core sire | 46 nm | | | | | 39 nm | | | | 24 nm |

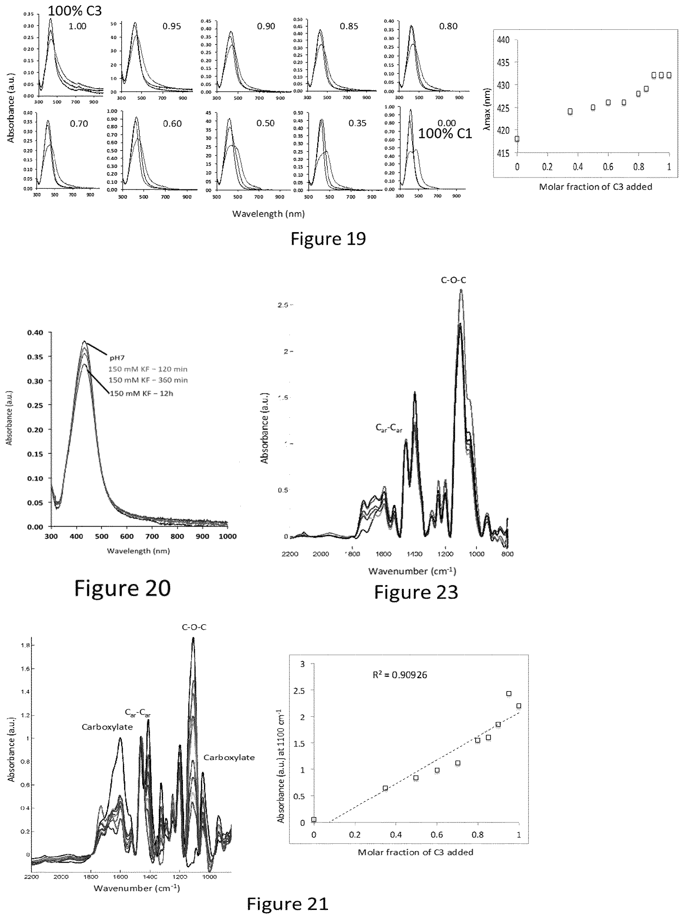

The **UV-visible spectra** of the Ag-C1C3 (Figure 19) show sharp absorption peaks between 432 and 418 nm, revealing well-dispersed silver nanoparticles with a narrow size distribution.
The final concentration of nanoparticles was estimated to be around 0.14-2.5 nM, based on the calculated extinction coefficient.

**Colloidal stability and chemical stability:** The chemical robustness of these hybrid Ag-C1C3 particles is demonstrated by exposing the particles to extreme conditions, either acidic (figure 19) or etching environment (figure 20). Figure 20 shows the etching resistance for the Ag-C1C3 in a 15:85 ratio. These particles can endure fluoride exposure during several hours without significant loss.

**The IR spectrum** of Ag-C1C3 (figure 21) clearly shows absorption peaks of the aromatics (1458 cm-1), the carboxylates (1620, 1350, 1050 cm-1) and of the polyethyleneglycol (PEG) chains (1100 cm-1) of the calixarene. For all calixarene ratios, the calixarenes are all well grafted onto the surface. Furthermore, the intensity of the PEG signal depends on the amount of C3 added during the synthesis (spectra normalized as described in example 7), thereby confirming that it is possible to (i) synthesize particles covered with a mixed layer of calixarenes and (ii) control the proportions of calixarenes within the mixed layer.

### Example 9: silver core nanomaterials coated with calix[n]arenes of type C2 and C3: Ag-C2C3

Nanoparticles with a pure silver core and coated with a mixture of calixarenes C3 and C2, in various ratio, were synthesized according to the general synthesis procedure 1.
The results are summarized in table 3 below.

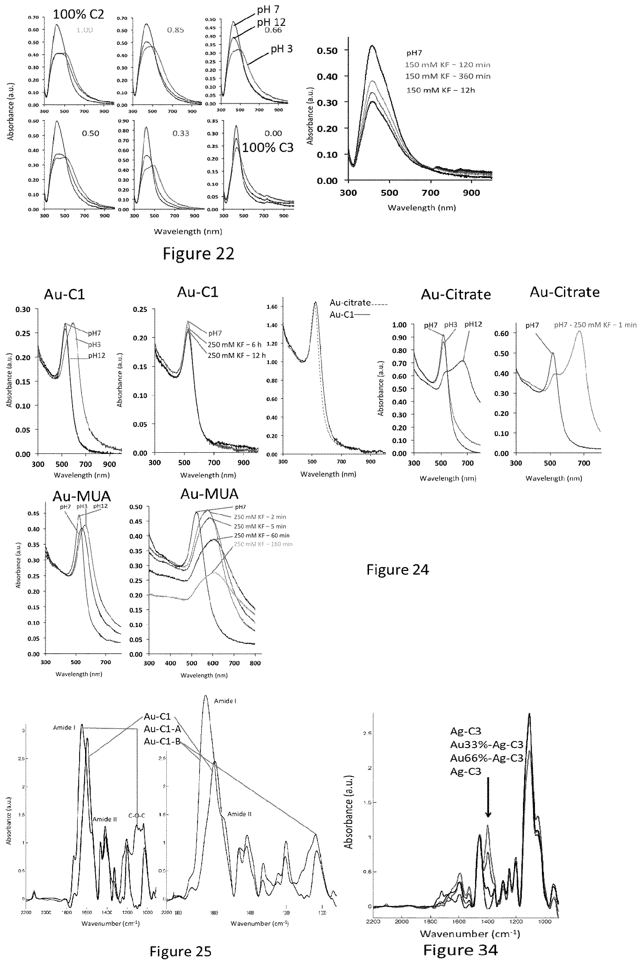

The **UV-visible spectra** of the Ag-C2C3 (Figure 22) show sharp absorption peaks between 424 and 432 nm, revealing well-dispersed silver nanoparticles with a narrow size distribution.
The final concentration of nanoparticles was estimated to be around 0.1-0.4 nM, based on the calculated extinction coefficient, depending on the amount of calixarene C2.

**Table 3.**

| Molar fraction of C2 | 1 (Ag-C2) | 0.85 | 0.66 | 0.5 | 0.33 | 0 (Ag-C3) |
|---|---|---|---|---|---|---|
| UV peak (nm) | 424 | | | | | 432 |
| Colloidal stability | Idem example 4 - | High resistance to acidic condition | Mild reversible aggregation | Reversible aggregation | Reversible aggregation | Idem example 6 - high resistance |
| Chemical stability | | No loss of particles | | | | |
| Core sire | 46 nm | | | | | 24 nm |

**Colloidal stability and chemical stability:** The chemical robustness of these hybrid Ag-C2C3 particles is demonstrated by exposing the particles to extreme conditions, either acidic or etching environment (figure 22). Figure 22 shows the etching resistance for the Ag-C2C3 in a 50:50 ratio. These particles are more resistant than commercially available particles.

**The IR spectrum** of Ag-C2C3 (figure 23) clearly shows absorption peaks of the aromatics (1458 cm⁻¹) and of the PEG chains (1100 cm⁻¹) of the calixarenes. For all calixarene ratios, the calixarenes are all well grafted onto the surface. Furthermore, the intensity of the PEG signal depends on the amount of C3 added during the synthesis (spectra normalized as described in example 7), thereby confirming that it is possible to (i) synthesize particles covered with a mixed layer of calixarenes and (ii) control the proportions of calixarenes within the mixed layer.

### Example 10: Gold core nanomaterials coated with calixarenes C1, C2 and/or C3.

Several calixarene coated gold nanomaterials or gold and silver coated nanomaterials were prepared using the same experimental procedure but starting with a gold salt: HAuCl₄.

Data regarding UV and IR spectrum, stability studies are gathered in Table 4.
Overall, it was possible to product gold nanoparticles coated with a stable and homogeneous layer of calixarenes (single type or mixtures). These particles demonstrate high colloidal and chemical robustness and possess the capacity to be post-functionalized. Nanomaterials with a core comprising gold and silver were prepared, the ratio of metal incorporated in the core matching the ratio of the oxidized metal used for the synthesis. Similarly, to the silver nanomaterials described above, mixed layers of calixarenes could also be successfully be prepared, with a good control of the layer composition.

**Table 4.**

| | Au-citrate | Aumercaptoundecanoic acid (Au-MUA) | Au-C1 | Au-C2 | Au-C3 | Au-C1C2 | Au-C1C2 | | | |
|---|---|---|---|---|---|---|---|---|---|---|
| **calixarene composition** | NA | NA | 100% C1 | 100% C2 | 100% C3 | 50% C1 | 66% C1 | | | |
| **detail of metallic composiition** | | | | | | | | | | |
| **Concentration (nM)** | | | 6.5 nM | 3.2 nM | 3.2 nM | | | | | |
| **extinction coeficient (L.mol^{- 1}.cm⁻¹)** | | | 4x10⁸ | 8x10⁸ | 8x10⁸ | | | | | |
| **absorption peak** | | | 525 nm | 533 nm | 530 nm | | | | | |
| **Stability** | | | | | | | | | | |
| **acidic conditions** | irreversible aggragation | reversible aggregation | reversible aggregation, absorption shifted to 590 nm | minor reversible aggregation | minor reversible aggregation | minor reversible aggregation | minor reversible aggregation | | | |
| **basic conditions** | | stable | stable | stable | stable | stable | stable | | | |
| **KF** | | degradation of particles | stable | stable | stable | stable | stable | | | |
| | | | | | | | | | | |
| **figure UV** | 24 | 24 | 24 | 26 | 28 | 26 | 26 | | | |
| **figure IR** | | | 25 | 27 | 30 | 27 | 27 | | | |
| **presence of calixarene on core surface** | | | aromatics (1458 cm⁻¹) | aromatics (1458 cm⁻¹) | aromatics (1458 cm⁻¹) | aromatics (1458 cm⁻¹) | aromatics (1458 cm⁻¹) | | | |
| | | | | | | carboxylates (1620, 1350, ( 1050 cm⁻¹) | carboxylates (1620, 1350, 1050 cm⁻¹) | | | |
| | | | carboxylates (1620, 1350, 1050 cm-1) | PEG chains (1100 cm⁻¹) | PEG chains (1100 cm-1) | PEG chains (1100 cm⁻¹) | PEG chains (1100 cm⁻¹) | | | |
| **Figure SEM** | | | | | 36 | | | | | |
| **particule size (SEM)** | | | 19 nm | | 22 nm | | | | | |
| **particle shape** | | | spherical | | spherical | | | | | |
| | | | | | | | | | | |
| **post fonctionalization** | | | NH2-PEG7-OCH3 (Figure 25, Au-C1-A) | | | | | | | |
| **post fonctionalization** | | | a peptide, whose sequence is AAPLSQETFSDLWKLL ((Figure 25, Au-C1-B) | | | | | | | |

| | Au-C2C3 | Au-C2C3 | Au-C2C3 | Au-C2C3 | AU-C1C3 | AU-C1C3 | AU-C1C3 | AU-C1C3 | AU-C1C3 | AU-C1C3 |
|---|---|---|---|---|---|---|---|---|---|---|
| **calixarene composition** | 85% C2 | 66% C2 | 50% C2 | 33% C2 | 90% C3 | 80% C3 | 70% C3 | 60% C3 | 50% C3 | 35% C3 |
| **detail of metallic composiition** | | | | | | | | | | |
| **Concentration (nM)** | 3.2 nM | 3.2 nM | 3.2 nM | 3.2 nM | | | | | | |
| **extinction coeficient (L.mol⁻** | 8x10⁸ | 8x10⁸ | 8x10⁸ | 8x10⁸ | | | | | | |
| **absorption peak** | | | | | | | | | | |
| **Stability** | | | | | | | | | | |
| **acidic conditions** | minor reversible aggregation | minor reversible aggregation | minor reversible aggregation | minor reversible aggregation | minor reversible aggregation | minor reversible aggregation | minor reversible aggregation | minor reversible aggregation | minor reversible aggregation | minor reversible aggregation |
| **basic conditions** | stable | stable | stable | stable | stable | stable | stable | stable | stable | stable |
| **KF** | stable | stable | stable | stable | stable | stable | stable | stable | stable | stable |
| | | | | | | | | | | |
| **figure UV** | 28 | 28 | 28 | 28 | 29 | 29 | 29 | 29 | 29 | 29 |
| **figure IR** | | | | | 30 | 30 | 30 | 30 | 30 | 30 |
| **presence of calixarene on core surface** | aromatics (1458 cm⁻¹) | aromatics (1458 cm⁻¹) | aromatics (1458 cm⁻¹) | aromatics (1458 cm⁻¹) | aromatics (1458 cm⁻¹) | aromatics (1458 cm⁻¹) | aromatics (1458 cm⁻¹) | aromatics (1458 cm⁻¹) | a romati cs (1458 cm⁻¹) | aromatics (1458 cm⁻¹) |
| | PEG chains (1100 cm⁻¹) | PEG chains (1100 cm⁻¹) | PEG chains (1100 cm⁻¹) | PEG chains (1100 cm⁻¹) | carboxylates (1620, 1350, 1050 cm⁻¹) | carboxylates (1620, 1350, 1050 cm⁻¹) | carboxylates (1620, 1350, 1050 cm⁻¹) | carboxylates (1620, 1350, 1050 cm⁻¹) | carboxylates (1620, 1350, 1050 cm⁻¹) | carboxylates (1620, 1350, 1050 cm⁻¹) |
| | | | | | PEG chains (1100 cm⁻¹) | PEG chains (1100 cm⁻¹) | PEG chains (1100 cm⁻¹) | PEG chains (1100 cm⁻¹) | PEG chains (1100 cm⁻¹) | PEG chains (1100 cm⁻¹) |
| | | | | | proportional | proportional | proportional | proportional | proportional | proportional |
| **particule size (SEM)** | | | | | | | | | | |
| **particle shape** | | | | | | | | | | |
| | | | | | | | | | | |
| **post fonctionalization** | | | | | | | | Cyanine dye with terminal amino group absorbing at 800 nm (Figure 31) | | |

| | Au-Ag-C1 | Au-Ag-C3 | Au-Ag-C3 | Au-Ag-C3 | | | | | | |
|---|---|---|---|---|---|---|---|---|---|---|
| **calixarene composition** | 100% C1 | 100% C3 | 100% C3 | 100% C3 | | | | | | |
| **detail of metallic composiition** | 50% Au - 50% Ag (molar) | 66% Au - 33% Ag (molar) | 33% Au - 66% Ag (molar) | 0% Au - 100 % Ag (molar) | | | | | | |
| **Concentration (nM)** | | | | 0,1 nM | | | | | | |
| **extinction coeficient (L.mol^{- 1}.cm⁻¹)** | | | | | | | | | | |
| **absorption peak** | 460 nm | ∼510 nm | ∼450 nm | 425 nm | | | | | | |
| **Stability** | | | | | | | | | | |
| **acidic conditions** | reversible aggregation, absorption shifted to 590 nm | | minor reversible aggregation | | | | | | | |
| **basic conditions** | stable | | stable | | | | | | | |
| **KF** | stable | | stable | | | | | | | |
| | | | | | | | | | | |
| **figure UV** | 30 | 33 | 33 | 33 | | | | | | |
| **figure IR** | 34 | 34 | 34 | 34 | | | | | | |
| **presence of calixarene on core surface** | aromatics (1458 cm⁻¹) | aromatics (1458 cm⁻¹) | aromatics (1458 cm⁻¹) | aromatics (1458 cm⁻¹) | | | | | | |
| | carboxylates (1620, 1350, 1050 cm⁻¹) | PEG chains (1100 cm-1) | PEG chains (1100 cm⁻¹) | PEG chains (1100 cm-1) | | | | | | |

### Example 10: Palladium, Platinum, Copper and or Iron oxide core nanomaterials coated with calixarenes C1, C2 and/or C3

Several calixarene-coated palladium, platinum, copper oxide and iron oxide nanomaterials were prepared using general procedure 1, starting from the appropriate salts: palladium chloride, platinum chloride, copper acetate or mix of Iron Chloride salts (33% FeCl₂ / 66% FeCl₃) NB
- For gold or alloys involving gold, the metallic salts solutions have to be heated at 60 °C for 20 minutes before the addition of the calixarenes and ascorbate;
- For iron nanoparticles, the sodium ascorbate solution is replaced by the same volume of a 2 M ammonia solution (NH₃ (aq)).
- For Iron nanoparticles, the final concentration of Iron is around 10 mM instead of 1 mM described in the general procedure.
- For copper nanoparticles, temperature higher than 70 °C and pH lower than 4.5 can lead to pure metallic copper core instead of copper oxide core.
- For copper nanoparticles, the half of the water added is replaced by acetonitrile.
- For copper nanoparticles, the ascorbate 15 mM added is replaced by a mixture composed of 50 % of ascorbate 15 mM and 50 % of NaBH₄ 150 mM.
- For platinum nanoparticles, platinum salt (PtCl₂) is dissolved in DMSO then this solution (20 mM) is diluted 2 times with pure water.
- For platinum nanoparticles, the calixarenes is added after the reductant, which are an equimolar mixture of ascorbate and NaBH₄.

Analytical data regarding the coated nanomaterials are gathered in Table 5.

**Table 5.**

| | Pd-C1 | Pd-C3 | Pt-C1 | Pt-C1-A | Cu-C1 | Fe-C1 |
|---|---|---|---|---|---|---|
| Variation to general procedure | | | | Solvent: DMSO Reducing agent: NaBH4 | | Reducing agent: NaOH |
| Absorption peak | | | | | 355 nm (emission at 405 nm; figure 41) | |
| Stability | | | | | | |
| Acidic condition | Reversible aggregation | | | | Reversible aggregation | |
| Basic condition | Stable | | | | Stable | |
| KF | Stable | | | | Stable | |
| Figure UV | | | | Same UV profile as Pt-C1 | 40 | |
| Figure IR | 35 | 37 | 39 | | 42 | 44 |
| Presence of calixarene on core surface | Aromatics (1458 cm⁻¹) | Aromatics (1458 cm⁻¹) | Aromatics (1458 cm⁻¹) | | Aromatics (1458 cm⁻¹) | Aromatics (1458 cm⁻¹) |
| | Carboxylates (1620, 1350, 1050 cm⁻¹) | PEG chains (1100 cm⁻¹) | Carboxylates (1620, 1350, 1050 cm⁻¹) | | Carboxylates (1620, 1350, 1050 cm⁻¹) | Carboxylates (1620, 1350 cm⁻¹) |
| | | | | | | |
| Particle size (SEM) | 45 nm | 80 nm | 40 nm | | 100 nm | 20 nm |
| Particle shape | Spherical (figure 36) | Spherical (figure 38) | Spherical | | Cubic shape (figure 43) | Spherical (figure 45) |

These experiments show the versatility of the method of the invention, to make metalic cores coated with a monolayer of calixarenes. The metallic cores can be a single metal, metal oxide or an alloy or metallic mixture. The ratio of the metals in the nanoparticle obtained is proportional to the ratio of oxidized metal introduced in the reaction mixture.

Depending on the metal, different shapes of nanoparticles can be obtained, in particular, nanospheres and nanocubes have been obtained.
The method of the invention works in water and in other solvents, like DMSO.
The method enables to obtain nanoparticles coated with several types of calixarenes, which is particularly convenient for introducing a variety of functionalities or post-functionalization possibilities. This can lead to advantageous properties in many different fields of activities, like microelectronics, IVD, biomedical applications, catalysis, nanoreactors formed by calixarenes at the surface of particles, ....

The repeatability of the experiments, and stability of the nanoparticles enable to extrapolate the results of the above experiments to all types of calixarenes, and many more metals.

## Claims

1. Method to synthesize metal-based nanomaterials coated with calix[n]arenes comprising the steps of:
- placing at least one oxidized metal with at least one calix[n]arene diazonium salt in the presence of a reducing agent in a solvent, and
- heating the reaction mixture to obtain a metal-based nanomaterial coated with calix[n]arenes.

2. Method according to claim 1, further comprising the step of adjusting the pH of the reaction mixture at a value comprised between 5 and 8.

3. Method according to one of claim 1 or 2, wherein the molar ratio of oxidized metal to the calix[n]arene diazonium salt is comprised between 10:1 and 1:10.

4. Method according to anyone of claims 1 to 3, wherein the oxidized metal comprises a metal oxide and/or a metal halide, nitrate, sulfate, carboxylate, triflate, sulfonate, phosphate, tosylate or a borate.

5. Method according to anyone of claims 1 to 4, wherein the metal of the oxidized metal is an alkali metal, an alkaline earth metal, a lanthanide, an actinide, a transition metal, a post transition metal, and/or a metalloid.

6. Method according to claim 5, wherein the metal is selected from the list comprising silver, palladium, gold, platinum, copper, nickel, zinc, cadmium, indium, lead, titanium, tantalum, silicon, aluminum or iron.

7. Method according to anyone of claims 1 to 6, wherein the calix[n]arene diazonium salt is a calix[4]arene diazonium salt.

8. Method according to anyone of claims 1 to 7 wherein the reducing agent is a hydride or an ascorbate salt.

9. Method according to anyone of claims 1 to 8, wherein the solvent is water.

10. Metal-based nanomaterials comprising a metallic core coated only with calix[n]arenes.

11. Metal-based nanomaterials according to claim 10, coated with an ultrathin layer of calix[n]arenes.

12. Metal-based nanomaterials according to one of claims 10 or 11, coated with an ultrathin layer of at least two types of calix[n]arenes.

13. Metal-based nanomaterials according to one of claims 10 to 12, wherein at least some grafted calix[n]arenes can be reacted with molecules or biomolecules.
